# EUROPÄISCHE PATENTANMELDUNG

(11) **EP 1 084 682 A2**
(43) Veröffentlichungstag der Anmeldung: **21.03.2001**
(21) Anmeldenummer: 00890254.6
(22) Anmeldetag: 22.08.2000
(51) Int. Cl.: A61C 8/00, A61B 17/66

(54) **Positioniervorrichtung für ein Kieferimplantat**

(30) Priorität: 15.09.1999 AT 158499
(71) Anmelder: Watzek, Georg, Dr., 1080 Wien (AT)
(72) Erfinder: Watzek, Georg, Dr., 1080 Wien (AT)
(74) Vertreter: Kliment, Peter

(57) **Zusammenfassung**

Positioniervorrichtung für ein Kieferimplantat, welches im Ober- oder Unterkiefer implantiert ist. Um eine Vorrichtung, welche es ermöglicht, ein Kieferimplantat ohne Knochentransplantation auch in Regionen mit lokalem Knochendefizit in das Kiefer zu implantieren, sowie das Kieferimplantat optimal im Kiefer auszurichten, so dass es in Bezug auf den Kiefer gerade nach oben oder unten steht ohne der Notwendigkeit einer Knochentransplantation oder Augmentation, zu schaffen, ist vorgesehen, dass ein mit dem Kieferimplantat (4) lösbar verbundener Aufsatz (24), welcher an seiner dem Kieferimplantat abgewandten Seite mit mindestens einem Gewinde versehen ist und mit diesem Gewinde in einer Öffnung (9) einer Stützplatte (10;20) frei drehbar gelagert ist, wobei die Stützplatte (10;20) fix mit den Zähnen oder anderen ortsfesten Punkten innerhalb des Mundes verbunden ist und auf der dem Kieferimplantat (4) abgewandten Seite der Stützplatte (10;20) eine Mutter (14;22;23) am Gewinde des Aufsatzes (24) angeordnet ist.

## Beschreibung

Die vorliegende Erfindung bezieht sich auf eine Positioniervorrichtung für ein Kieferimplantat gemäß Oberbegriff des Anspruchs 1.

Wird ein ausgefallener Zahn im menschlichen Kiefer nicht ersetzt, bildet sich in der entstandenen Zahnlücke der Kieferknochen zurück, wodurch ein sogenannter Substanzdefekt im Kiefer entsteht. Soll bei einem solchen, bereits vorhandenen Knochendefizit ein Zahnersatz geschaffen werden, so kann auf Wunsch des Patienten ein Kieferimplantat in das menschliche Kiefer (Ober- oder Unterkierfer) implantiert werden.

Bislang ist es erforderlich, die fehlende Knochensubstanz von einer anderen Stelle des Körpers an die Implantationsstelle zu transferieren. Nach der Knochentransplantation wird das Kieferimplantat in den Kieferknochen eingeschraubt und für eine gewisse Zeitspanne (ca. 3 Monate) in Ruhe belassen, wodurch der Kieferknochen an das Kieferimplantat heranwachsen kann. Nach Ablauf dieser Zeitspanne ist das Kieferimplantat vollkommen (abgesehen von der Oberseite) vom Kieferknochen umwachsen und an seiner Oberseite vom Zahnfleisch umgeben. Das Zahnfleisch wird daraufhin an der Implantatoberseite aufgeschnitten, beiseite geschoben und am Innengewinde des Kieferimplantats ein Verlängerungsstück und in weiterer Folge an diesem der eigentliche Zahnersatz angeschraubt.

Nachteil dieses Art der Implementierung eines Kieferimplantats ist jedoch die Tatsache, dass auf jeden Fall eine Knochentransplantation von einer beliebigen Stelle des Körpers in den Kiefer erforderlich ist. Dies ist jedoch mit einem erheblichen Aufwand und auch, wie jede Operation, mit einem gewissen Risiko verbunden. Auch bereitet die Ausrichtung des Kieferimplantats im Kieferknochen nach dem Stand der Technik noch gewisse Schwierigkeiten.

Dies hat zur Folge, das die Wunde unschön verheilt und sich starke Narben bilden können.

Ziel der vorliegenden Erfindung ist daher eine Vorrichtung, welche es ermöglicht, ein Kieferimplantat ohne Knochentransplantation auch in Regionen mit lokalem Knochendefizit in das Kiefer zu implantieren, sowie das Kieferimplantat optimal im Kiefer auszurichten, so dass es in Bezug auf den Kiefer gerade nach oben oder unten steht ohne der Notwendigkeit einer Knochentransplantation oder Augmentation.

Erfindungsgemäß wird dies durch die kennzeichnenden Merkmale des Anspruchs 1 erreicht.

Dadurch ist es möglich, durch Anziehen der Mutter, das Kieferimplantat in die jeweilige, der Drehachse des Gewindes entsprechende Richtung, zu bewegen. Als Vorbedingung dafür ist es erforderlich, das Kieferimplantat von dem es umgebenden Knochen freizuschneiden, so dass es sich samt anhaftenden Knochen sowohl horizontal als auch vertikal frei bewegen kann. Ist dies geschehen, so kann durch die erfindungsgemäße Vorrichtung die Tatsache genutzt werden, dass sich aufgrund der aus dem Anziehen der Mutter resultierenden Aufweitung des Spaltes zwischen den freigeschnittenen Knochenteilen, neuer Knochen nachbildet (Distraktionsosteogenese).

Je nach Anordnung des Gewindes kann das Kieferimplantat dabei in verschiedenste Richtungen bewegt werden (abhängig von der Schraubrichtung) und demgemäß auch die Knochennachbildung an verschiedensten Stellen erreicht werden.

Durch das kennzeichnende Merkmal des Anspruchs 2 kann der erfindungsgemäße Aufsatz auf einfache Art und Weise an jedes im Handel erhältliche Kieferimplantat aufgeschraubt werden. Dadurch ist unabhängig vom Fabrikat des Kieferimplantats ein korrektes Positionieren dieses möglich.

Durch das kennzeichnende Merkmal des Anspruchs 3 kann die erfindungsgemäße Positioniervorrichtung auch für jene Kieferimplantate verwendet werden, die ein Aufstecken der Verlängerung und des eigentlichen Zahnersatzes ermöglichen.

Durch die Merkmale des Anspruchs 4 kann der Basisteil des Aufsatzes an den jeweiligen Einsatzzweck angepasst werden. So ist es beispielsweise möglich, je nach Anzahl der verwendeten Gewindestifte den Basisteil kleiner oder größer auszuführen und mit mehreren Bohrungen oder weniger zu versehen. Auch ist es auf diese Art und Weise sehr einfach möglich jenen Teil des Aufsatzes, der das Gewinde umfasst (also beispielsweise der Gewindestift) auszutauschen, falls sich das Gewinde in folge der Benutzung als nicht mehr präzise genug erweist.

Durch die Merkmale des Anspruchs 5 können mehrere Gewindestifte gleichzeitig durch eine einzige Stützplatte gesteckt werden, wobei durch die Schlitzform der Stützplatte die Gewindestifte der Bewegung des Kieferimplantats folgen können.

Durch die Merkmale der Ansprüche 6 bis 9 ist es möglich das Kieferimplantat in die jeweilige gewünschte Richtung zu verschieben, wobei je nach Lage des Kieferimplantats der translatorischen Bewegung eine Kippbewegung überlagert wird.

Durch die Merkmale des Anspruchs 10 kann durch einfaches Drehen des Gewindestiftes ein Kippen des Kieferimplantats erreicht werden. Der Gewindestift stützt sich dabei auf der Widerstandsplatte ab, welche fix und unbeweglich montiert ist. Da sich die Widerstandsplatte nicht bewegen kann, wird die durch die Schraubung auftretende Kraft auf den Gewindestift und in weiterer Folge auf den mit dem Kieferimplantat verbundenen Aufsatz übertragen, welcher sich dadurch entgegen der Schraubrichtung bewegt.

Im folgenden erfolgt nun eine detaillierte Beschreibung der Erfindung anhand von Zeichnungen. Dabei zeigen
- Fig. 1: eine Ansicht eines menschlichen Unterkiefers
- Fig. 2: einen Schnitt durch ein menschliches Unterkiefer entlang der Linie II-II in Fig. 1 mit einer installierten erfindungsgemäßen Vorrichtung
- Fig. 3: einen Schnitt durch ein menschliches Unterkiefer entlang der Linien III-III in Fig. 1 mit einer installierten erfindungsgemäßen Vorrichtung
- Fig. 4: einen Grund - und Aufriß einer Stützplatte
- Fig. 5: einen Schnitt durch ein menschliches Unterkiefer entlang der Linie III-III mit einer installierten erfindungsgemäßen Kippvorrichtung

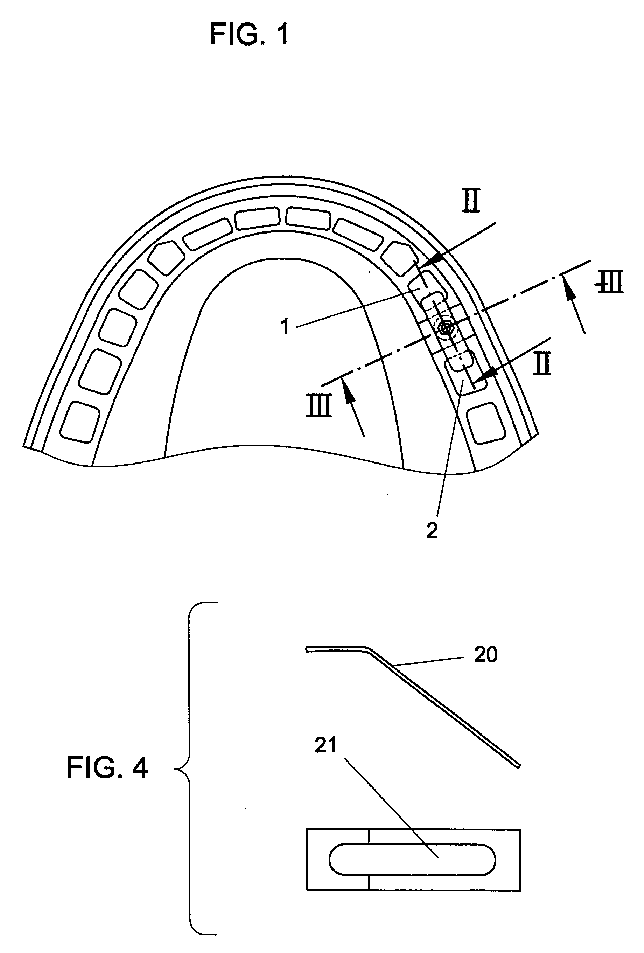

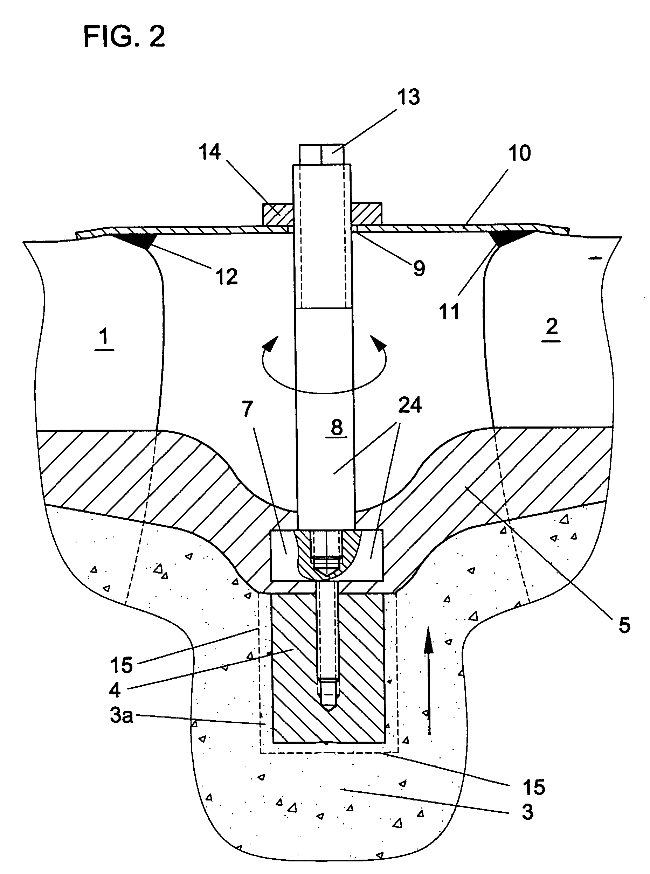

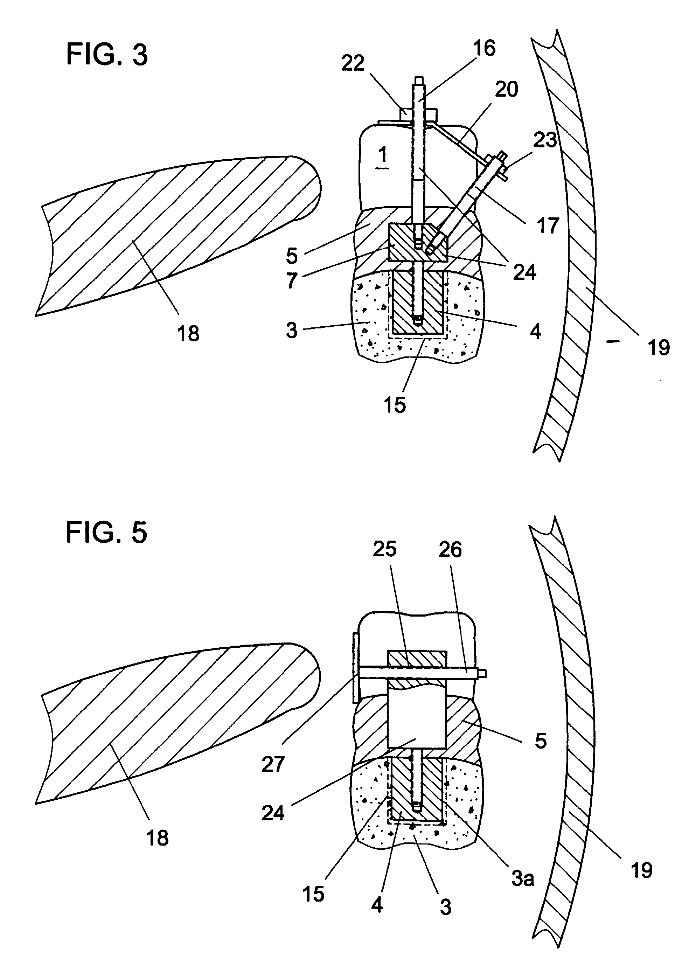

Fig. 1 zeigt einen Grundriß eines menschlichen Unterkiefers sowie die Schnittführungen für die Figuren 2 und 3.

In Fig. 2 ist eine Lücke zwischen zwei Zähnen 1,2 sichtbar. Eine solche Lücke entsteht beispielsweise durch Zahnausfall. Wird der Zahn nicht ersetzt bildet sich an dieser Stelle der Kieferknochen 3 zurück.

In den Kieferknochen 3 wird nunmehr ohne vorherige Knochentransplantation ein Kieferimplantat 4 geschraubt. Nach einer gewissen Ruhezeit, in der Regel 3 Monate) ist der Kieferknochen 3 an das Kieferimplantat 4 herangewachsen.

Durch das Zahnfleisch 5 wird nunmehr der erfindungsgemäße Aufsatz 24 am Kieferimplantat 4 befestigt. In der Regel wird dies durch eine Schraubung erfolgen, es ist jedoch auch vorstellbar, dass Steckverbindungen zum Einsatz kommen.

Im vorliegenden Ausführungsbeispiel (Fig.2) besteht der erfindungsgemäße Aufsatz aus einem Basisteil 7, der eine Bohrung mit Innengewinde aufweist, in welche ein Gewindestift 8 eingeschraubt ist. Es ist jedoch auch möglich den Aufsatz 24 aus einem Stück zu fertigen. Der Gewindestift 8 ragt dabei jedenfalls durch eine Öffnung 9 in einer Stützplatte 10. Die Stützplatte 10 ist an mehreren Stellen 11,12 an anderen Zähnen 1,2 bzw. an anderen Kieferimplantaten (nicht gezeichnet) befestigt, vorzugsweise geklebt.

Der Gewindestift 8 weist in seinem oberen Endbereich einen Sechskant 13 auf, um in den Basisteil 7 eingeschraubt werden zu können. Weiters ist eine Mutter 14 auf den oberen, das Gewinde tragenden Bereich des Gewindestiftes 8 aufgeschraubt.

Um die erfindungsgemäße Positioniervorrichtung anwenden zu können, ist es erforderlich das Kieferimplantat 4 vorerst entlang der Linie 15 freizuschneiden. Dies deswegen, um einen schon länger bekannten Effekt ausnutzen zu können, nämlich das sich Knochensubstanz bei sukzessiver Dehnung in dem durch die Dehnung entstehen Spalt nachbildet.

Im vorliegenden Fall kann also nach Anbringung der erfindungsgemäßen Vorrichtung 6 durch Anziehen der Mutter 14 die gesamte Einheit Gewindestift 8, Basisteil 7 und Kieferimplantat 4 samt jenem Knochenteil 3a, welcher freigeschnitten das Kieferimplantat 4 umgibt, hochgezogen werden.

Der Anzug der Mutter soll dabei so stark erfolgen, dass die dadurch hervorgerufene Bewegung zwischen 0,5 mm/Tag und 1,5 mm/Tag vorzugsweise 1 mm/Tag beträgt.

Auf diese Art und Weise kann der oben beschriebene Effekt ausgenutzt werden und es bildet sich so in dem entstandenen Spalt der beiden entlang der Schnittlinie 15 getrennten Kieferknochenteile 3,3a neue Knochensubstanz nach. Dadurch wird das Kieferimplantat samt umgebenden Knochen sukzessive nach oben befördert.

Fig. 3 zeigt die gleichzeitige Verwendung zweier Gewindestifte 16,17, welche in den Basisteil 7 eingeschraubt sind. Dabei kann eine Bewegung des Kieferimplantats 4 in eine Richtung bewirkt werden, die einer Überlagerung der Kraftwirkungsrichtungen der Gewindestifte 16,17 entspricht. Das Kieferimplantat 4 kann somit nicht nur nach oben gezogen werden, sondern auch in eine oder mehrere - je nach Anzahl der Gewindestifte - alternative Richtungen, beispielsweise in Richtung zur Zunge 18 und in Richtung zur Wange/Lippe 19 (Fig.3), je nachdem wo weitere Gewindestifte angeordnet sind.

Die Gewindestifte 16,17 sind dabei allesamt durch eine Öffnung 21 in der Stützplatte 20 frei drehbar durchgesteckt. Die Öffnung 21 ist dabei vorzugsweise ein Schlitz (Fig. 4). Dadurch blockieren die einzelnen Gewindestifte 16,17 nicht Bewegungen die durch das Anziehen der Muttern 22,23 an jeweils anderen Gewindestiften bewirkt werden.

Die beliebige Anordnung der Gewindestifte 16,17 im Basisteil 7 ermöglicht somit dieser beliebigen Anordnung entsprechende Dehnungen des Knochenschnitts/-spalts 15 und in weiterer Folge eine Nachbildung der Knochensubstanz des Kieferknochens 3,3a an den gewünschten Positionen.

Eine weitere Möglichkeit der Bewegung des Kieferimplantats zeigt Fig. 5. Dabei ist ein Aufsatz 24 der im wesentlichen dem Basisteil 7 aus Fig. 2,3 entspricht auf das Kieferimplantat 4 aufgeschraubt. Der Aufsatz 24 weist dabei mindestens eine Bohrung 25 auf, durch welche ein Gewindestift 26 geschraubt ist. Der Gewindestift 26 stützt sich in weiterer folge auf eine Widerstandsplatte 27. Diese ist wiederum mit anderen Zähnen bzw. mit anderen Kieferimplantaten fix verbunden, vorzugsweise geklebt und somit fix angeordnet.

Der Gewindestift 26 kann nun solange in den Aufsatz 24 eingeschraubt werden bis er an der in Schraubrichtung gesehen gegenüberliegenden Seite an die Widerstandsplatte 27 anstößt. Da diese fix angeordnet ist muß bei weiterer Schraubbewegung der Gewindestift 26 in die entgegengesetzte Richtung zur Schraubbewegung ausweichen. Auf diese Art und Weise kann die Einheit Aufsatz 24-Kieferimplantat 4 gekippt werden. Gekippt deswegen, weil der Aufsatz 24 im Zahnfleisch 5 angeordnet ist, das Kieferimplantat 4 hingegen im Kieferknochen 3 bzw. 3a., der wesentlich mehr Widerstand bietet und somit weniger nachgibt als das Zahnfleisch 5. Auch kann durch gezielte Schnittführung des Schnitts 15 das durch die Schraubung entstehende Kippmoment gefördert werden.

Eine perfekte Positionierung des Kieferimplantats 4 ist somit unter Neubildung von Knochensubstanz durch die erfindungsgemäße Vorrichtung leicht möglich.

Die oben beschriebenen Ausführungsbeispiele zeigen lediglich eine von vielen möglichen Anordnungsmöglichkeiten und Varianten. Die Ausbildung des Aufsatzes insbesondere des Basisteiles unterliegt keinerlei Einschränkungen und kann je nach Erfordernis in seiner Form den Gegebenheiten angepasst werden.

Die Ausführungsbeispiele wurden in ihrer Anordnung weiters anhand der Positionierung eines Kieferimplantats im Unterkiefer gezeigt. Selbstverständlich kann die gleiche erfindungsgemäße Positioniereinrichtung auf gleiche Art und Weise zur Positionierung eines Kieferimplantats im Oberkiefer verwendet werden.

## Patentansprüche

1. Positioniervorrichtung für ein Kieferimplantat (4), welches im Ober- oder Unterkiefer implantiert ist, **dadurch gekennzeichnet, dass** ein mit dem Kieferimplantat (4) lösbar verbundener Aufsatz (24) vorgesehen ist, welcher an seiner dem Kieferimplantat (4) abgewandten Seite mit mindestens einem Gewinde versehen ist und mit diesem Gewinde durch eine Öffnung (9) einer Stützplatte (10;20) frei drehbar durchgesteckt ist, wobei die Stützplatte (10;20) fix mit den Zähnen (1,2) oder anderen ortsfesten Punkten innerhalb des Mundes verbunden ist und auf der dem Kieferimplantat (4) abgewandten Seite der Stützplatte (10;20) eine Mutter (14;22;23) am Gewinde des Aufsatzes (24) angeordnet ist, welche gegen die Stützplatte (10;20) anziehbar ist.

2. Positioniervorrichtung nach Anspruch 1, **dadurch gekennzeichnet, dass** der Aufsatz (24) in das Kieferimplantat (4) eingeschraubt ist.

3. Positioniervorrichtung nach Anspruch 1, **dadurch gekennzeichnet, dass** der Aufsatz (24) in das Kieferimplantat (4) eingesteckt ist.

4. Positioniervorrichtung nach einem der Ansprüche 1 bis 3, **dadurch gekennzeichnet, dass** der Aufsatz (27) einen Basisteil (7) umfaßt, welcher mindestens eine Gewindebohrung aufweist und in diese Gewindebohrung ein Gewindestift (8;16;17) eingeschraubt ist.

5. Positioniervorrichtung nach einem der Ansprüche 1 bis 4, **dadurch gekennzeichnet, dass** die Öffnung (9) der Stützplatte (10;20) im wesentlichen Schlitzform hat, wobei die Breite des Schlitzes (21) mindestens dem Durchmesser des Gewindestiftes (8;16;17) entspricht.

6. Positioniervorrichtung nach einem der Ansprüche 1 bis 5, **dadurch gekennzeichnet, dass** die Stützplatte (10;20) im Mund oberhalb des Kieferimplantats (4) angeordnet ist.

7. Positioniervorrichtung nach einem der Ansprüche 1 bis 6, **dadurch gekennzeichnet, dass** die Stützplatte (10;20) im Mund unterhalb des Kieferimplantats (4) angeordnet ist.

8. Positioniervorrichtung nach einem der Ansprüche 1 bis 7, **dadurch gekennzeichnet, dass** die Stützplatte (10;20) im Mund zwischen Zunge (18) und Kieferknochen (3) angeordnet ist.

9. Positioniervorrichtung nach einem der Ansprüche 1 bis 8, **dadurch gekennzeichnet, dass** die Stützplatte (10;20) im Mund zwischen Wange bzw. Lippe (19) und Kiefer (3) angeordnet ist.

10. Positioniervorrichtung für ein Kieferimplantat, welches im Ober- oder Unterkiefer implantiert ist, **dadurch gekennzeichnet, dass** ein mit dem Kieferimplantat lösbar verbundener Aufsatz (24) mindestens eine Bohrung mit Gewinde aufweist, durch welche ein Gewindestift (26) schraubbar ist, der mit seiner einen Stirnseite an einer in Schraubrichtung gesehen hinter dem Aufsatz (24) angeordneten Widerstandsplatte (27) anliegt, wobei die Widerstandsplatte (27) fix mit den Zähnen oder anderen ortsfesten Punkten innerhalb des Mundes verbunden ist.
